# EUROPÄISCHE PATENTANMELDUNG

(11) **EP 4 455 703 A1**
(43) Veröffentlichungstag der Anmeldung: **30.10.2024**
(21) Anmeldenummer: 23169447.2
(22) Anmeldetag: 24.04.2023
(51) Int. Cl.: G01R 33/28

(54) **MAGNETRESONANZVORRICHTUNG MIT EINER PATIENTENKOMMUNIKATIONSEINHEIT**

(71) Anmelder: Siemens Healthineers AG, 91301 Forchheim (DE)
(72) Erfinder: Ruf, Marcel, 91094 Langensendelbach (DE); Karl, Harald, 90765 Fürth (DE)
(74) Vertreter: Siemens Healthineers Patent Attorneys

(57) **Zusammenfassung**

Die Erfindung geht aus von einer Magnetresonanzvorrichtung mit einer Magneteinheit, einen von der Magneteinheit zumindest teilweise umgebenen Patientenaufnahmebereich, wobei der Patientenaufnahmebereich zu einer Aufnahme eines Patienten für eine Magnetresonanzuntersuchung ausgebildet ist, und einer Patientenkommunikationseinheit, wobei die Patientenkommunikationseinheit eine Displayeinheit mit einem E-Paper-Display umfasst, wobei das E-Paper-Display innerhalb des Patientenaufnahmebereichs angeordnet ist.

## Beschreibung

Die vorliegende Erfindung betrifft eine Magnetresonanzvorrichtung mit einer Magneteinheit, einen von der Magneteinheit zumindest teilweise umgebenen Patientenaufnahmebereich, wobei der Patientenaufnahmebereich zu einer Aufnahme eines Patienten für eine Magnetresonanzuntersuchung ausgebildet ist, und einer Patientenkommunikationseinheit.

Unabhängig vom grammatikalischen Geschlecht eines bestimmten Begriffes sind Personen mit männlicher, weiblicher oder anderer Geschlechteridentität mit umfasst.

Während einer Magnetresonanzuntersuchung befindet sich der Patient innerhalb eines Untersuchungsraums, innerhalb dessen die Magnetresonanzvorrichtung angeordnet ist. Dabei ist der Patient innerhalb des Patientenaufnahmebereichs für die Magnetresonanzuntersuchung positioniert. Ein die Magnetresonanzuntersuchung betreuendes medizinisches Bedienpersonal, beispielsweise ein Arzt, dagegen hält sich während der Magnetresonanzuntersuchung in einem Kontrollraum auf. Von diesem Kontrollraum aus kann das medizinische Bedienpersonal die Magnetresonanzuntersuchung steuern und überwachen.

Für eine Kommunikation mit dem Patienten während der Magnetresonanzuntersuchung weist die Magnetresonanzvorrichtung für gewöhnlich eine Patientenkommunikationseinheit auf. Die Patientenkommunikationseinheit dient dazu, dem Patienten während der Magnetresonanzuntersuchung Informationen und/oder Anweisungen zu übermitteln. Beispielsweise können hierbei dem Patienten für die Magnetresonanzuntersuchung erforderliche Atemanweisungen übermittelt werden, wie insbesondere Atemanhalte-Anweisungen und/oder Breathhold-Signale, die dem Patienten mitteilen, wann er die Luft anhalten muss. Dies ist vor allem bei Thorax-Untersuchungen wichtig. Zudem kann mittels der Patientenkommunikationseinheit dem Patienten auch eine verbleibende Scandauer und/oder Restuntersuchungszeit mitgeteilt werden.

Bisher werden derartige Informationen und/oder Anweisungen mittels einer akustischen Ausgabeeinheit, beispielsweise in Form von Kopfhörern, die der Patient während der Magnetresonanzuntersuchung trägt, an dem Patienten übermittelt.

Eine Anordnung eines herkömmlichen Displays und/oder eines Monitors innerhalb des Patientenaufnahmebereichs einer Magnetresonanzvorrichtung dagegen weist viele Schwierigkeiten auf. So kann insbesondere die Magnetresonanzuntersuchung durch Störsignale einer Elektronik des Displays gestört werden, was zu unerwünschten Artefakten in den erfassten Bilddaten und/oder zu Messwiederholungen führen kann. Des Weiteren wird dabei das Display innerhalb eines sehr starken Magnetfelds, beispielsweise von 1,5 T oder 3T, positioniert, was auch zu Störungen der Elektronik des Displays selbst führen kann und damit zu eine Störung der Patientenkommunikation. Eine Störung der Patientenkommunikation kann sich wiederum negativ auf den Patienten auswirken, so dass unter Umständen auch einzelne Messungen der Magnetresonanzuntersuchung wiederholt werden müssen. Eine weitere Schwierigkeit bei einer Anbringung eines herkömmlichen Displays stellt die Stromversorgung des Displays dar, was wiederum zu Störungen der Magnetresonanzuntersuchung führen kann.

Der vorliegenden Erfindung liegt insbesondere die Aufgabe zugrunde, eine visuelle Patientenkommunikation auf einfache Art und Weise während einer Magnetresonanzuntersuchung an einem Patienten bereitzustellen. Die Aufgabe wird durch die Merkmale des unabhängigen Anspruchs gelöst. Vorteilhafte Ausgestaltungen sind in den Unteransprüchen beschrieben.

Die Erfindung geht aus von einer Magnetresonanzvorrichtung mit einer Magneteinheit, einen von der Magneteinheit zumindest teilweise umgebenen Patientenaufnahmebereich, wobei der Patientenaufnahmebereich zu einer Aufnahme eines Patienten für eine Magnetresonanzuntersuchung ausgebildet ist, und einer Patientenkommunikationseinheit. Erfindungsgemäß umfasst die Patientenkommunikationseinheit eine Displayeinheit mit einem E-Paper-Display.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Zudem kann die erfindungsgemäße Magnetresonanzvorrichtung zusammen mit einer PET-Vorrichtung (Positron-Emission-Tomographie-Vorrichtung) auch in ein kombiniertes Magnetresonanz-PET-System integriert sein.

Die Magnetresonanzvorrichtung umfasst bevorzugt die Magneteinheit zur Erfassung der medizinischen und/oder diagnostischen Bilddaten. Hierbei umfasst die Magneteinheit einen Grundmagneten, eine Gradientenspuleneinheit und eine Hochfrequenzantenneneinheit. Die Hochfrequenzantenneneinheit ist hierbei fest innerhalb der Magneteinheit angeordnet.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das Gradienten-System ist zu einer Erzeugung von Magnetfeldgradienten, die für eine Ortskodierung während einer Bildgebung verwendet werden, ausgebildet. Die Hochfrequenzantenneneinheit ist zu einer Aussendung von Hochfrequenzpulsen und/oder Anregungspulsen zur Generierung von Magnetresonanzsignalen ausgebildet.

Für eine Magnetresonanzuntersuchung wird der Patient, insbesondere der zu untersuchende Bereich des Patienten, innerhalb des Patientenaufnahmebereichs der Magnetresonanzvorrichtung positioniert. Innerhalb des Patientenaufnahmebereichs ist bevorzugt das Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Zu einem Einfahren und/oder Positionieren des Patienten, insbesondere des zu untersuchenden Bereichs des Patienten, innerhalb des Patientenaufnahmebereichs umfasst die Magnetresonanzvorrichtung eine Patientenlagerungsvorrichtung. Die Patientenlagerungsvorrichtung weist dabei einen Patiententisch auf, wobei der Patiententisch relativ zur Magneteinheit bewegbar ausgebildet ist. Bevorzugt ist hierbei der Patiententisch in Längsrichtung des Patiententisch und/oder in Längsrichtung des Patientenaufnahmebereichs innerhalb des Patientenaufnahmebereichs bewegbar ausgebildet, um den Patienten in einer Untersuchungsposition innerhalb des Patientenaufnahmebereich zu positionieren.

Die Patientenkommunikationseinheit ist zu einer Kommunikation zwischen einem Benutzer, insbesondere dem die Magnetresonanzuntersuchung betreuenden Bedienpersonal, und dem Patienten während einer Magnetresonanzuntersuchung ausgebildet. Dazu weist die Patientenkommunikationseinheit auf Patientenseite die Displayeinheit auf, die innerhalb eines Untersuchungsraums, der die Magneteinheit umfasst, angeordnet ist. Zudem kann die Patientenkommunikationseinheit eine zweite Kommunikationseinheit umfassen, die auf Benutzerseite, insbesondere innerhalb eines Kontrollraums, von dem aus die Magnetresonanzuntersuchung von einem medizinischen Bedienpersonal gesteuert und/oder überwacht wird, angeordnet ist. Beispielsweise kann die zweite Kommunikationseinheit eine Eingabeeinheit zu einer Eingabe von Informationen und/oder Anweisungen, die an den Patienten übermittelt werden sollen, umfassen.

Mittels der Displayeinheit sollen dem Patienten Anweisungen und/oder Informationen, insbesondere untersuchungsrelevante Anweisungen und/oder Informationen, während der Magnetresonanzuntersuchung übermittelt und/oder mitgeteilt werden. Beispielsweise kann derart dem Patienten eine verbleibende Restuntersuchungszeit mitgeteilt werden.

Die Displayeinheit umfasst ein E-Paper-Display und/oder EInk-Display. Ein E-Paper-Display und/oder EInk-Display weist die Eigenschaft auf, dass es Licht wie normales Papier reflektieren. Insbesondere umfasst ein E-Paper-Display und/oder EInk-Display die Eigenschaft auf, dass es eine passive Anzeige, insbesondere eine nichtleuchtende Anzeige, umfasst. Ein E-Paper-Display und/oder EInk-Display kann auch als reflektives Display bezeichnet werden.

Bevorzugt ist die Displayeinheit derart innerhalb des Untersuchungsraums angeordnet, dass ein Patient in einer Untersuchungsposition eine direkte Sicht auf die Displayeinheit, insbesondere auf das E-Paper-Display und/oder EInk-Display, hat. Dabei kann die Displayeinheit an der den Patientenaufnahmebereich umgebenden Umhausung oder auch an dem Patiententisch angeordnet sein.

Derartige Displays, insbesondere E-Paper-Displays und/oder EInk-Displays, weisen den Vorteil auf, dass angezeigte Informationen und/oder Anweisungen dauerhaft ohne Energiezufuhr angezeigt und/oder dargestellt werden können. Insbesondere benötigen derartige Displays, insbesondere E-Paper-Displays und/oder EInk-Displays, nur bei einer Änderung eines Anzeigeninhalts und/oder eines Darstellungsinhalts Energie, so dass eine benötigte Energie sehr gering ist. Beispielsweise benötigt ein E-Paper-Display und/oder EInk-Display einen Strom im Bereich von wenigen 100 mA. Insbesondere kann hierdurch während einer Magnetresonanzuntersuchung auf eine ständig bestromte Elektronik der Displayeinheit vorteilhaft verzichtet werden. Insbesondere kann derart ein Energiebedarf sehr geringgehalten werden und damit auch eine unerwünschte Interaktion zwischen der Displayeinheit und der Magneteinheit während einer Magnetresonanzuntersuchung geringgehalten und/oder verhindert werden. Beispielsweise kann die Displayeinheit dazu ausgebildet sein, nur in Messpausen, insbesondere in Pausen zwischen zwei auszuspielenden Hochfrequenzpulsen, einen Anzeigeninhalt des E-Paper-Displays und/oder EInk-Displays zu ändern. Insbesondere können derart unerwünschte Störungen der Displayeinheit und/oder der Magnetresonanzdatenerfassung reduziert und/oder verhindert werden. Derart kann auf konstruktiv einfache Art und Weise eine visuelle Information dem Patienten während der Magnetresonanzuntersuchung bereitgestellt werden.

Zudem können derartige Displayeinheiten, insbesondere das E-Paper-Display und/oder EInk-Display, besonders kompakt und platzsparend ausgebildet werden. Insbesondere kann derart ein für den Patienten zur Verfügung stehender Aufenthaltsraum innerhalb des Patientenaufnahmebereich durch eine Anordnung der Displayeinheit innerhalb des Patientenaufnahmebereichs erhalten bleiben.

In einer vorteilhaften Weiterbildung der erfindungsgemäßen Magnetresonanzvorrichtung kann es vorgesehen sein, dass die Displayeinheit ein Antennenelement aufweist, wobei das Antennenelement zu einer Energiegewinnung während einer Magnetresonanzuntersuchung ausgebildet ist. Bevorzugt ist das Antennenelement zu einem Empfang von elektromagnetischen Wellen ausgebildet, wobei aus den empfangenen elektromagnetischen Wellen Energie, insbesondere elektrische Energie, gewonnen wird. Insbesondere ist die Displayeinheit und damit auch das Antennenelement während einer Magnetresonanzuntersuchung innerhalb des Patientenaufnahmebereichs angeordnet, wobei das Antennenelement bevorzugt zum Empfang von Hochfrequenzpulsen der Magneteinheit zu einer Energiegewinnung ausgebildet ist.

Diese Ausgestaltung der Erfindung weist den Vorteil auf, dass die Displayeinheit autark während einer Magnetresonanzuntersuchung innerhalb des Patientenaufnahmebereichs betrieben werden kann. Insbesondere kann derart auf eine zusätzliche Stromversorgung der Displayeinheit, beispielsweise durch zusätzliche Stromkabel und/oder mittels Batterien, vorteilhaft verzichtet werden. Insbesondere kann derart die Displayeinheit drahtlos und/oder kabellos betrieben werden. Ein weiterer Vorteil ist, dass auch auf ein Überwachen eines Ladezustands von Batterien verzichtet werden kann und damit die Displayeinheit innerhalb des Patientenaufnahmebereichs stets einsatzbereit ist. Zudem kann hierdurch eine flexible Positionierung der Displayeinheit bereitgestellt werden, die an eine Patientenpositionierung angepasst werden kann.

In einer vorteilhaften Weiterbildung der erfindungsgemäßen Magnetresonanzvorrichtung kann es vorgesehen sein, dass das Antennenelement an eine Sendefrequenz einer Hochfrequenzantenneneinheit der Magneteinheit, insbesondere während einer Magnetresonanzuntersuchung, angepasst ist. Derart kann auf konstruktiv einfache Art und Weise eine Energiegewinnung ermöglicht werden und zudem auf eine externe Energiezufuhr verzichtet werden. Des Weiteren steht hierdurch der Displayeinheit, insbesondere dem E-Paper-Display und/oder EInk-Display, stets während einer Magnetresonanzuntersuchung eine elektrische Energie zur Verfügung. Zudem können Störungen der Displayeinheit, insbesondere des E-Paper-Displays und/oder EInk-Displays, während einer Magnetresonanzuntersuchung verhindert werden.

In einer vorteilhaften Weiterbildung der erfindungsgemäßen Magnetresonanzvorrichtung kann es vorgesehen sein, dass die Displayeinheit ein Energiespeicherelement aufweist. Das Energiespeicherelement umfasst bevorzugt Akkus, wie beispielsweise Kondensatoren. Diese Ausgestaltung der Erfindung weist den Vorteil auf, dass elektrische Energie, insbesondere mittels des Antennenelements gewonnene Energie, für eine Verwendung durch die Displayeinheit gespeichert werden kann. Insbesondere kann derart auch elektrische Energie für die Displayeinheit, insbesondere das E-Paper-Display und/oder EInk-Display, zur Verfügung stehen, auch wenn die Magnetresonanzuntersuchung noch nicht gestartet ist, wie beispielsweise nach einem Einfahren des Patienten in den Patientenaufnahmebereich. Insbesondere weist die Ausgestaltung der Displayeinheit mit dem Energiespeicherelement den Vorteil auf, dass ein konstanter Strom und/oder eine konstante Spannung für einen Betrieb der Displayeinheit zur Verfügung gestellt werden kann. Zudem werden hierdurch auch bei der Energiegewinnung auftretende Spannungsspitzen nicht direkt an eine Elektronik der Displayeinheit weitergegen, sondern es tritt eine glättende Wirkung durch das Energiespeicherelement, insbesondere der Kondensatoren, ein.

In einer vorteilhaften Weiterbildung der erfindungsgemäßen Magnetresonanzvorrichtung kann es vorgesehen sein, dass die Displayeinheit ein erstes Funkmodul umfasst, das zu einem Empfangen von Steuerdaten ausgebildet ist. Das erste Funkmodul umfasst bevorzugt eine Funkschnittstelle, die zu einem Empfangen von Funkdaten, insbesondere Steuerdaten, ausgebildet ist. Bevorzugt erfolgt mittels der Steuerdaten eine Steuerung einer Anzeige auf dem E-Paper-Display und/oder EInk-Display. Beispielsweise können die Steuerdaten eine anzuzeigende Information enthalten, die auf dem E-Paper-Display und/oder EInk-Display angezeigt wird. Zudem können die Steuerdaten auch ein Anzeigen einer anzuzeigenden Information auf dem E-Paper-Display und/oder EInk-Display auslösen, wobei die anzuzeigende Information bereits auf der Displayeinheit gespeichert ist. Das Funkmodul korrespondiert hierzu bevorzugt mit einem zweiten Funkmodul, das die Steuerdaten an das erste Funkmodul sendet. Bevorzugt befindet sich das zweite Funkmodul außerhalb des Patientenaufnahmebereichs. Bevorzugt erfolgt eine Kommunikation zwischen den beiden Funkmodulen mit einer Frequenz, die außerhalb eine Sendespektrums und/oder eines Empfangsspektrums der Magnetresonanzvorrichtung angeordnet ist.

Diese Ausgestaltung der Erfindung weist den Vorteil auf, dass eine einfache Datenübertragung von einem Kontrollraum und/oder von einem Benutzer an den Patienten erfolgen kann. Zudem kann derart die Datenübertragung an die Displayeinheit kabellos und/oder drahtlos erfolgen und auf eine zusätzliche Datenleitung vorteilhaft verzichtet werden.

In einer vorteilhaften Weiterbildung der erfindungsgemäßen Magnetresonanzvorrichtung kann es vorgesehen sein, dass die Patientenkommunikationseinheit eine Steuereinheit und ein zweites Funkmodul aufweist, wobei das zweite Funkmodul zu einer Datenübertragung mit dem ersten Funkmodul ausgebildet ist, wobei die Steuereinheit zusammen mit dem zweiten Funkmodul außerhalb des Patientenaufnahmebereichs angeordnet ist.

Bevorzugt ist die Steuereinheit zu einer Generierung von Steuersignalen, die an das erste Funkmodul und damit an die Displayeinheit übertragen werden sollen, ausgebildet. Die Steuereinheit umfasst zumindest ein Rechenmodul und/oder einen Prozessor. So ist insbesondere die Steuereinheit dazu ausgebildet, computerlesbare Instruktionen auszuführen. Insbesondere kann die Steuereinheit eine Speichereinheit umfassen, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen, um eine Datenübertragung zwischen den beiden Funkmodulen zu steuern und/oder um Steuerdaten für eine Steuerung einer Anzeige auf der Displayeinheit zu generieren.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Auch hier kann der Vorteil erreicht werden, dass eine einfache Datenübertragung, insbesondere eine kabellose und/oder drahtlose Datenübertragung, von einem Kontrollraum und/oder vom Benutzer an den Patienten erfolgen kann.

In einer vorteilhaften Weiterbildung der erfindungsgemäßen Magnetresonanzvorrichtung kann es vorgesehen sein, dass die Displayeinheit eine Platine mit einem Elektronikmodul umfasst, wobei auf der Platine das erste Funkmodul angeordnet ist. Bevorzugt ist die Platine mit dem E-Paper-Display und/oder EInk-Display zu einer Datenübertragung und/oder einer Energieübertragung verbunden. Hierdurch kann eine besonders kompakte und platzsparende Anordnung des ersten Funkmoduls innerhalb der Displayeinheit erreicht werden.

In einer vorteilhaften Weiterbildung der erfindungsgemäßen Magnetresonanzvorrichtung kann es vorgesehen sein, dass die Magneteinheit eine den Patientenaufnahmebereich umgebende Umhausung aufweist, an der die Displayeinheit angeordnet ist. Die den Patientenaufnahmebereich umgebende Umhausung umfasst beispielsweise ein Tragrohr, an dem auf einer dem Patientenaufnahmebereich abgewandten Seite die Hochfrequenzantenneneinheit angeordnet ist. Bevorzugt ist hierbei die Displayeinheit an einer einer Lagerungsfläche zur Lagerung des Patienten gegenüberliegenden Oberfläche der den Patientenaufnahmebereich umgebenden Umhausung angeordnet. Durch die Erfindung kann eine direkte Sichtbarkeit des E-Paper-Displays und/oder EInk-Displays für den Patienten erreicht werden.

In einer vorteilhaften Weiterbildung der erfindungsgemäßen Magnetresonanzvorrichtung kann es vorgesehen sein, dass die Displayeinheit zumindest ein Befestigungselement aufweist, wobei das zumindest eine Befestigungselement zu einer lösbaren Befestigung zwischen der Displayeinheit und der den Patientenaufnahmebereich umgebenden Umhausung ausgebildet ist. Derart kann vorteilhaft eine Position der Displayeinheit an eine Position des Patienten, insbesondere an eine Untersuchungsposition des Patienten, angepasst werden. Insbesondere kann dabei die Displayeinheit an unterschiedlichen Positionen an der den Patientenaufnahmebereich umgebenden Umhausung angeordnet werden.

Das zumindest eine Befestigungselement kann beispielswiese zu einem Ankleben der Displayeinheit an der den Patientenaufnahmebereich umgebenden Umhausung ausgebildet sein. Dabei kann das zumindest eine Befestigungselement beispielsweise als doppelseitiges Klebeband ausgebildet sein. Zudem können auch an der den Patientenaufnahmebereich umgebenden Umhausung definierte Positionen mit einem Befestigungselement versehen sein, die ein Einrasten und/oder Einhängen und/oder Verklemmen der Displayeinheit an der den Patientenaufnahmebereich umgebenden Umhausung ermöglichen. Je nach Untersuchungsposition des Patienten kann dabei die Displayeinheit an einer dieser Positionen befestigt werden.

In einer vorteilhaften Weiterbildung der erfindungsgemäßen Magnetresonanzvorrichtung kann es vorgesehen sein, dass die Magnetresonanzvorrichtung einen Patiententisch mit zumindest einem abnehmbaren Befestigungselement umfasst, wobei das zumindest eine Befestigungselement derart am Patiententisch befestigbar ist, dass die Displayeinheit bei der Anordnung und/oder Befestigung am Befestigungselement in einem Sichtfeld des Patienten angeordnet ist. Insbesondere ist hierbei das E-Paper-Display und/oder EInk-Display im Sichtfeld des Patienten angeordnet. Beispielsweise kann das zumindest eine Befestigungselement ein bogenförmiges Halteelement umfassen, dass an dem Patiententisch, insbesondere in Kopfnähe des Patienten am Patiententisch, befestigbar ist, so dass die Displayeinheit im Sichtfeld des Patienten positioniert und/oder angeordnet werden kann. Bevorzugt ist dabei die Displayeinheit zusammen mit dem Patiententisch in den Patientenaufnahmebereich einfahrbar. Derart befindet sich die Displayeinheit stets im Sichtfeld des Patienten, unabhängig von dessen Position innerhalb des Patientenaufnahmebereichs. Insbesondere kann derart eine uneingeschränkte Sichtbarkeit des E-Paper-Displays und/oder EInk-Displays für den Patienten bereitgestellt werden.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung mit einer Patientenkommunikationseinheit in einer schematischen Darstellung,
- Fig. 2: eine Displayeinheit der Patientenkommunikationseinheit und
- Fig. 3: eine alternative Anordnung der Displayeinheit an einem Patiententisch.

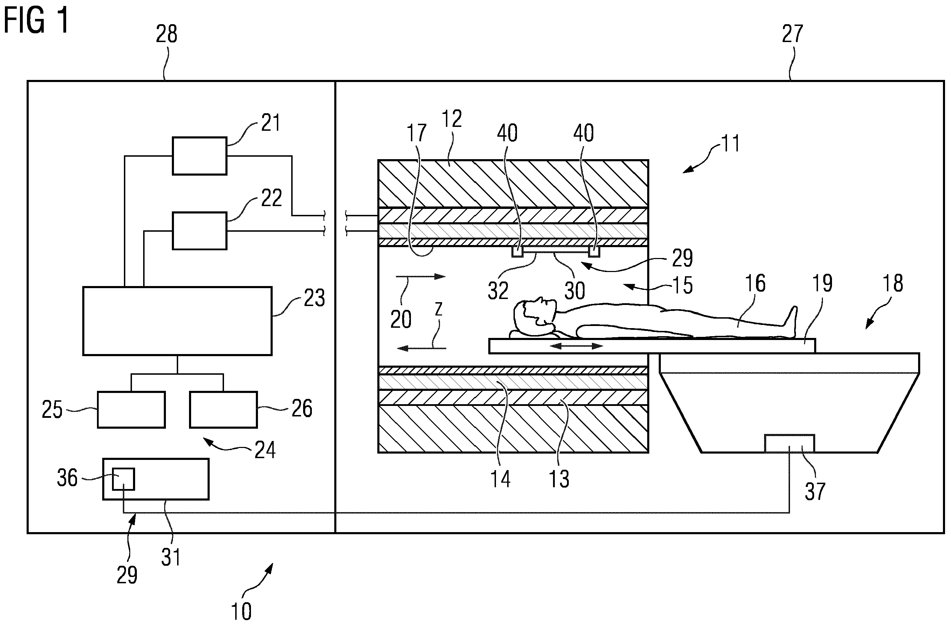

In der Fig. 1 ist eine Magnetresonanzvorrichtung 10 schematisch dargestellt. Die Magnetresonanzvorrichtung 10 umfasst eine Magneteinheit 11 mit einem Grundmagneten 12, einer Gradientenspuleneinheit 13 und einer Hochfrequenzantenneneinheit 14. Zudem weist die Magnetresonanzvorrichtung 10 einen Patientenaufnahmebereich 15 auf zu einer Aufnahme eines Patienten 16. Der Patientenaufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 15 jederzeit denkbar. Die Magneteinheit 11 weist zudem eine den Patientenaufnahmebereich 15 umgebende Umhausung 17 auf. Die den Patientenaufnahmebereich 15 umgebende Umhausung 17 umfasst bevorzugt ein Tragrohr, das den Patientenaufnahmebereich 15 zylinderförmig umgibt. An einer dem Patientenaufnahmebereich 15 abgewandten Seite des Tragrohrs ist bevorzugt die Hochfrequenzantenneneinheit 14 der Magneteinheit 11 angeordnet.

Der Patient 16 kann mittels einer Patientenlagerungsvorrichtung 18 der Magnetresonanzvorrichtung 10 in den Patientenaufnahmebereich 15 geschoben und/oder gefahren werden. Die Patientenlagerungsvorrichtung 18 weist hierzu einen innerhalb des Patientenaufnahmebereichs 15 bewegbar ausgestalteten Patiententisch 19 auf. Insbesondere ist hierbei der Patiententisch 19 in Richtung einer Längserstreckung des Patientenaufnahmebereichs 15 und/oder in z-Richtung bewegbar gelagert.

Der Grundmagnet 12 der Magneteinheit 11 umfasst einen supraleitenden Grundmagneten 12 zu einem Erzeugen eines starken und insbesondere konstanten Grundmagnetfelds 20. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 21 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 20 einstellt, ausgebildet. Die Hochfrequenzantenneneinheit 14 wird von einer Hochfrequenzantennensteuereinheit 22 der Magnetresonanzvorrichtung 10 gesteuert und strahlt hochfrequente Magnetresonanzsequenzen in den Patientenaufnahmebereich 15 der Magnetresonanzvorrichtung 10 ein.

Zu einer Steuerung des Grundmagneten 12, der Gradientensteuereinheit 21 und zur Steuerung der Hochfrequenzantennensteuereinheit 22 weist die Magnetresonanzvorrichtung 10 eine Systemsteuereinheit 23 auf. Die Systemsteuereinheit 23 steuert zentral die Magnetresonanzvorrichtung 10, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Darstellungseinheit 25, beispielsweise auf zumindest einem Monitor, der Benutzerschnittstelle 24 für ein medizinisches Bedienpersonal angezeigt werden. Weiterhin weist die Benutzerschnittstelle 24 eine Eingabeeinheit 26 auf, mittels der Informationen und/oder Parameter während eines Messvorgangs von dem medizinischen Bedienpersonal eingegeben werden können.

Die Magneteinheit 11 der Magnetresonanzvorrichtung 10 ist zusammen mit der Patientenlagerungsvorrichtung 18 innerhalb eines Untersuchungsraums 27 angeordnet. Die Systemsteuereinheit 23 dagegen ist zusammen mit der Benutzerschnittstelle 24 innerhalb eines Kontrollraums 28 angeordnet. Der Kontrollraum 28 ist getrennt von dem Untersuchungsraum 27 ausgebildet. Insbesondere ist der Untersuchungsraum 27 hinsichtlich einer Hochfrequenzstrahlung von dem Kontrollraum 28 abgeschirmt. Während einer Magnetresonanzuntersuchung befindet sich der Patient 16 innerhalb des Untersuchungsraums 27, dagegen befindet sich das medizinische Bedienpersonal innerhalb des Kontrollraums 28.

Für eine Kommunikation zwischen dem Bediener, insbesondere einem medizinischen Bedienpersonal, und dem Patienten 16 während der Magnetresonanzuntersuchung weist die Magnetresonanzvorrichtung 10 eine Patientenkommunikationseinheit 29 auf. Mittels der Patientenkommunikationseinheit 29 sollen dem Patienten 16 während der Magnetresonanzuntersuchung Informationen und/oder Anweisungen, insbesondere untersuchungsrelevante Informationen und/oder Anweisungen, übermittelt und/oder mitgeteilt werden, wie beispielsweise eine verbleibende Untersuchungszeit und/oder Atemkommandos usw. Die Patientenkommunikationseinheit 29 weist eine Displayeinheit 30 auf, die innerhalb des Untersuchungsraums 27, insbesondere innerhalb des Patientenaufnahmebereichs 15 und damit in der Nähe zum Patienten 16, angeordnet ist. Des Weiteren weist die Patientenkommunikationseinheit 29 auch eine benutzerseitige Kommunikationseinheit 31 auf, die im Kontrollraum 28 angeordnet ist.

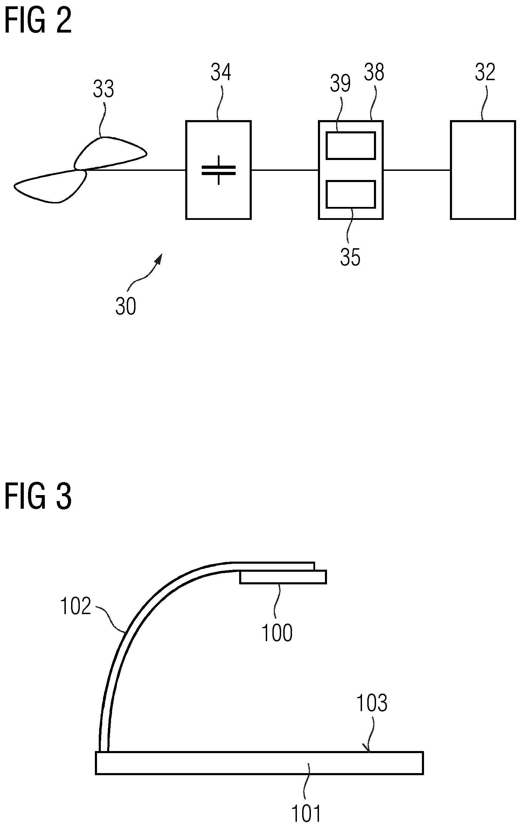

Die Displayeinheit 30 ist in Fig. 2 näher dargestellt. Die Displayeinheit 30 umfasst ein E-Paper-Display 32 und/oder EInk-Display, das innerhalb des Patientenaufnahmebereichs 15 angeordnet ist. Die Displayeinheit 30 weist weiterhin ein Antennenelement 33 auf, das zu einer Energiegewinnung, insbesondere zur Gewinnung von elektrischer Energie, während einer Magnetresonanzuntersuchung ausgebildet ist. Insbesondere ist das Antennenelement 33 dazu ausgebildet, eine elektrische Energie aus den elektromagnetischen Wellen und/oder der elektromagnetischen Strahlung der ausgesandten Sendepulse und/oder Hochfrequenzpulse der Hochfrequenzantenneneinheit 14 zu gewinnen. Hierbei ist bevorzugt das Antennenelement 33 an eine Sendefrequenz der Hochfrequenzantenneneinheit 14 angepasst ausgebildet.

Die Displayeinheit 30 weist des Weiteren ein Energiespeicherelement 34 auf. Das Energiespeicherelement 34 ist zu einem Speichern von elektrischer Energie ausgebildet und umfasst einen wiederaufladbaren Akku, in dem die von dem Antennenelement 33 gewonnene elektrische Energie gespeichert werden kann und bei Bedarf an das E-Paper-Display 32 und/oder EInk-Display und/oder eine Elektronik der Displayeinheit 30 abgegeben werden kann. Der wiederaufladbare Akku umfasst dabei bevorzugt Kondensatoren zur Speicherung der elektrischen Energie.

Weiterhin weist die Displayeinheit 30 ein erstes Funkmodul 35 der Patientenkommunikationseinheit 29 auf, das zu einem Empfangen von Steuerdaten zur Steuerung des E-Paper-Displays 32 und/oder EInk-Displays ausgebildet ist. Die Patientenkommunikationseinheit 29 weist zudem eine Steuereinheit 36 und ein zweites Funkmodul 37 auf, wobei die Steuereinheit 36 von der benutzerseitigen Kommunikationseinheit 31 innerhalb des Kontrollraums 28 umfasst sind. Das zweite Funkmodul 37 ist dabei innerhalb des Untersuchungsraums 27 angeordnet, beispielsweise an einer Basiseinheit der Patientenlagerungsvorrichtung 18, wobei mittels einer Datenverbindung das zweite Funkmodul 37 mit der Steuereinheit 36 verbunden ist. Das erste Funkmodul 35 und das zweite Funkmodul 37 sind zu einer Datenübertragung, insbesondere zu einer Übertragung und/oder einen Austausch von Steuerdaten zur Steuerung des E-Paper-Displays 32 und/oder EInk-Displays, zwischen der benutzerseitigen Kommunikationseinheit 31 und der Displayeinheit 30 ausgebildet. Mittels der Steuereinheit 36 werden die Steuerdaten zur Steuerung einer Anzeige auf dem E-Paper-Display 32 und/oder EInk-Display generiert. Bevorzugt erfolgt eine Kommunikation zwischen den beiden Funkmodulen 35, 37 mittels einer Funkfrequenz, die außerhalb eine Sendespektrums und/oder eines Empfangsspektrums der Hochfrequenzantenneneinheit 14 angeordnet ist.

Die Displayeinheit 30 weist des Weiteren eine Platine 38 mit einem Elektronikmodul 39 auf. Auf der Platine 38 ist des Weiteren auch das erste Funkmodul 35 angeordnet.

Die Displayeinheit 30 ist an der den Patientenaufnahmebereich 15 umgebenden Umhausung 17 angeordnet (Fig. 1). Die Displayeinheit 30 weist hierzu zumindest ein Befestigungselement 40 auf, das zu einer lösbaren Befestigung zwischen der Displayeinheit 30 und der den Patientenaufnahmebereich 15 umgebenden Umhausung 17, insbesondere dem Tragrohr, ausgebildet ist. Für eine derartige Anordnung der Displayeinheit 30 an der den Patientenaufnahmebereich 15 umgebenden Umhausung 17 kann die Displayeinheit 30 auch zwei oder mehr Befestigungselemente 40 aufweisen.

Dabei kann das zumindest eine Befestigungselement 40 ein Klebeelement umfassen, das zu einem Ankleben der Displayeinheit 30 an der der Patientenaufnahmebereich 15 umgebenden Umhausung 17 ausgebildet ist. Bevorzugt bewirkt das Klebeelement eine lösbare Klebeverbindung zwischen der Displayeinheit 30 und der den Patientenaufnahmebereich 15 umgebenden Umhausung 17.

Alternativ hierzu kann die den Patientenaufnahmebereich 15 umgebende Umhausung 17 auch definierte Aufnahmeplätze zur Aufnahme und/oder Anordnung der Displayeinheit 30 umfassen. Bevorzugt kann hierzu die Displayeinheit 30 ein Klemmelement und/oder ein Rastelement für eine Klemmverbindung und/oder Rastverbindung mit der den Patientenaufnahmebereich 15 umgebenden Umhausung 17 aufweisen. Zudem sind weitere, dem Fachmann als sinnvoll erscheinende Befestigungselemente zur Befestigung und/oder Anordnung der Displayeinheit 30 an der den Patientenaufnahmebereich 15 umgebenden Umhausung 17 jederzeit denkbar. Insbesondere ist hierbei die Displayeinheit 30 auch lösbar in den definierten Aufnahmeplätzen angeordnet, so dass je nach Untersuchungsposition des Patienten 16 die Displayeinheit 30 in einer der Aufnahmeplätze positioniert werden kann und der Patienten 16 eine direkte Sicht auf die Displayeinheit 30 hat.

In Fig. 3 ist ein alternatives Ausführungsbeispiel der Magnetresonanzvorrichtung dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 und 2, wobei bezüglich gleichbleibender Bauteile, Merkmale und Funktionen auf die Beschreibung des Ausführungsbeispiels in den Fig. 1 und 2 verwiesen wird.

Die Magnetresonanzvorrichtung in Fig. 3 unterscheidet sich von der Magnetresonanzvorrichtung 10 in Fig. 1 in einer Anordnung der Displayeinheit 100. Die Displayeinheit 100 ist im vorliegenden Ausführungsbeispiel am Patiententisch 101 angeordnet, wobei in Fig. 3 nur der Patiententisch 101 mit der Displayeinheit 100 in einer Schnittdarstellung zu sehen ist. Hierzu weist der Patiententisch 101 ein abnehmbares Befestigungselement 102 auf, das sich in Querrichtung des Patiententisches 101 bogenförmig über den Patiententisch 101, insbesondere eine Lagerungsfläche 103 des Patiententischs 101 zur Lagerung des Patienten 16, erstreckt und/oder spannt. An diesem Befestigungselement 102 ist die Displayeinheit 30 abnehmbar angeordnet. Dabei ist die Displayeinheit 30 derart an dem Befestigungselement 102 angeordnet, dass die Displayeinheit 30 direkt im Sichtfeld des Patienten 16 angeordnet ist. Bei einem Bewegen des Patiententischs 101, beispielsweise bei einem Einfahren des Patiententischs 101 in den Patientenaufnahmebereich 15, befindet sich somit die Displayeinheit 30 immer direkt im Sichtfeld des Patienten 16.

Eine weitere Ausgestaltung der Displayeinheit 30 entspricht den obigen Ausführungen der Beschreibung zu Fig. 2, auf die hiermit verwiesen wird.

Eine weitere Ausgestaltung der Magnetresonanzvorrichtung entspricht zudem den Ausführungen der Beschreibung zu Fig. 1, auf die hiermit verwiesen wird.

Die dargestellten Magnetresonanzvorrichtungen 10, 100 können selbstverständlich weitere Komponenten umfassen, die Magnetresonanzvorrichtungen 10, 100 gewöhnlich aufweisen. Eine allgemeine Funktionsweise einer Magnetresonanzvorrichtung 10, 100 ist zudem dem Fachmann bekannt, so dass auf eine detaillierte Beschreibung der weiteren Komponenten verzichtet wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung mit einer Magneteinheit, einen von der Magneteinheit zumindest teilweise umgebenen Patientenaufnahmebereich, wobei der Patientenaufnahmebereich zu einer Aufnahme eines Patienten für eine Magnetresonanzuntersuchung ausgebildet ist, und einer Patientenkommunikationseinheit,
**dadurch gekennzeichnet, dass** die Patientenkommunikationseinheit eine Displayeinheit mit einem E-Paper-Display umfasst.

2. Magnetresonanzvorrichtung nach Anspruch 1,
**dadurch gekennzeichnet, dass** die Displayeinheit ein Antennenelement aufweist, wobei das Antennenelement zu einer Energiegewinnung während einer Magnetresonanzuntersuchung ausgebildet ist.

3. Magnetresonanzvorrichtung nach Anspruch 2,
**dadurch gekennzeichnet, dass** das Antennenelement an eine Sendefrequenz einer Hochfrequenzantenneneinheit der Magneteinheit angepasst ist.

4. Magnetresonanzvorrichtung nach einem der vorhergehenden Ansprüche,
**dadurch gekennzeichnet, dass** die Displayeinheit ein Energiespeicherelement aufweist.

5. Magnetresonanzvorrichtung nach einem der vorhergehenden Ansprüche,
**dadurch gekennzeichnet, dass** die Displayeinheit ein erstes Funkmodul umfasst, das zu einem Empfangen von Steuerdaten ausgebildet ist.

6. Magnetresonanzvorrichtung nach Anspruch 5,
**dadurch gekennzeichnet, dass** die Patientenkommunikationseinheit eine Steuereinheit und ein zweites Funkmodul aufweist, wobei das zweite Funkmodul zu einer Datenübertragung mit dem ersten Funkmodul ausgebildet ist, wobei die Steuereinheit mit dem zweiten Funkmodul außerhalb des Patientenaufnahmebereichs angeordnet ist.

7. Magnetresonanzvorrichtung nach einem der vorhergehenden Ansprüche,
**dadurch gekennzeichnet, dass** die Displayeinheit eine Platine mit einem Elektronikmodul umfasst, wobei auf der Platine das erste Funkmodul angeordnet ist.

8. Magnetresonanzvorrichtung nach einem der vorhergehenden Ansprüche,
**dadurch gekennzeichnet, dass** die Magneteinheit eine den Patientenaufnahmebereich umgebende Umhausung aufweist, an der die Displayeinheit angeordnet ist.

9. Magnetresonanzvorrichtung nach Anspruch 8,
**dadurch gekennzeichnet, dass** die Displayeinheit zumindest ein Befestigungselement aufweist, wobei das zumindest eine Befestigungselement zu einer lösbaren Befestigung zwischen der Displayeinheit und der den Patientenaufnahmebereich umgebenden Umhausung ausgebildet ist.

10. Magnetresonanzvorrichtung nach einem der vorhergehenden Ansprüche,
**gekennzeichnet durch** einen Patiententisch mit zumindest einem abnehmbaren Befestigungselement, wobei das zumindest eine Befestigungselement derart am Patiententisch befestigbar ist, dass die Displayeinheit bei der Anordnung am Befestigungselement in einem Sichtfeld des Patienten angeordnet ist.
